# EUROPEAN PATENT APPLICATION

(11) **EP 3 792 242 A1**
(43) Date of publication of application: **17.03.2021**
(21) Application number: 19799142.5
(22) Date of filing: 10.05.2019
(51) Int. Cl.: C07C 47/21, A23L 2/02, A23L 27/00, A23L 27/20, A61K 8/35, A61Q 11/00, A61Q 13/00, C11B 9/00

(54) **DIENAL COMPOUND AND PERFUME COMPOSITION**

(30) Priority: 11.05.2018 JP 2018092059
(71) Applicant: Takasago International Corporation, Tokyo 144-8721 (JP)
(72) Inventor: TAKAHASHI, Tomoya, Hiratsuka-shi, Kanagawa 254-0003 (JP); KAWARAYA, Akihiro, Hiratsuka-shi, Kanagawa 254-0003 (JP); ZAIZEN, Kyoko, Hiratsuka-shi, Kanagawa 254-0003 (JP); INABA, Teruhiko, Hiratsuka-shi, Kanagawa 254-0003 (JP)
(74) Representative: Hoffmann Eitle
(86) International application number: PCT/JP2019/018740
(87) International publication number: WO 2019/216412

(57) **Abstract**

The present invention relates to: a novel dienal compound which has an aromatic odor useful for use as a compound flavor raw material or the like; and a flavor composition containing the dienal compound. The dienal compound according to the present invention is characterized by being represented by general formula (1): 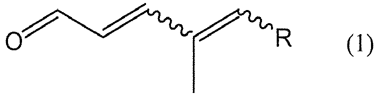 (wherein R represents an alkyl group having 8 to 10 carbon atoms; and a wavy line represents a cis form, a trans form, or a mixture of a cis form and a trans form).

## Description

### TECHNICAL FIELD

The present invention relates to a dienal compound and a flavor composition which are useful for use as a compound flavor raw material or the like.

### BACKGROUND ART

Recently, since palatability of consumers has become diverse, regarding flavors to be used for food and drink, etc., a flavor rich in fruitiness, and natural feeling and a flavor differentiated from other ones have been strongly desired. Combinations of conventional flavor raw materials cannot sufficiently satisfy such demands, and a novel flavor material has been strongly desired. For the purpose of imparting or enhancing natural feeling, various techniques have been proposed. For example, methods for adding 7,9,11-dodecatriene-4-one, 6,10-undecadiene-3-one or 6,8-undecadiene-3-one described in Patent Literature 1, 2(E),8(Z)-tetradecadiene-1-al described in Patent Literature 2, cis-4,5-epoxy-2E-decenal described in Patent Literature 3 or the like are disclosed.

### CITATION LIST

### Patent Literature

Patent Literature 1: Japanese Laid-Open Patent Publication No. 2010-83913
Patent Literature 2: Japanese Laid-Open Patent Publication No. 2016-124833
Patent Literature 3: Japanese Laid-Open Patent Publication No. 2005-82771

### SUMMARY OF INVENTION

### TECHNICAL PROBLEM

The problem to be solved by the present invention is to provide a novel dienal compound, which has an aromatic odor useful for use as a compound flavor raw material or the like, and a flavor composition containing the dienal compound.

### SOLUTION TO PROBLEM

The present inventors synthesized various compounds and examined aromatic odors thereof, and found that a dienal compound having a chain structure with a specific carbon number has an excellent aromatic odor.

The present invention includes the below-described contents.
[1] A compound represented by general formula (1): 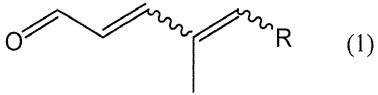 wherein: R represents an alkyl group having 8 to 10 carbon atoms; and a wavy line represents a cis form, a trans form, or a mixture of a cis form and a trans form.
[2] The compound of item [1], wherein R is an alkyl group having 8 carbon atoms.
[3] The compound of item [1] or [2], wherein position 2 is a trans form.
[4] The compound of item [3], wherein position 4 is a cis form.
[5] A flavor composition, comprising the compound of any one of items [1] to [4].
[6] The flavor composition of item [5], wherein the flavor composition is a fruit-like flavor composition.
[7] The flavor composition of item [6], wherein the fruit is a citrus fruit.
[8] The flavor composition of item [6] or [7], wherein the flavor composition is the flavor composition for a food or drink.
[9] The flavor composition of item [8], wherein said food or drink is a beverage.
[10] The flavor composition of item [9], wherein the beverage is a citrus-based beverage.
[11] A product selected from the group consisting of a food or drink, an oral composition and a tobacco product, comprising the compound of any one of items [1] to [4].
[12] A product selected from the group consisting of a food or drink, an oral composition and a tobacco product, comprising the flavor composition of any one of items [5] to [10].

### ADVANTAGEOUS EFFECTS OF INVENTION

According to the present invention, it is possible to provide a novel dienal compound, which has a useful odor for use as a flavor raw material or the like, and a flavor composition containing the dienal compound.

### DESCRIPTION OF EMBODIMENTS

Hereinafter, the present invention will be described in detail.

Regarding the dienal compound of the present invention represented by general formula (1): 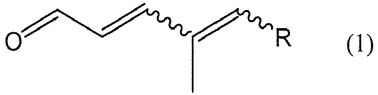 wherein: R represents an alkyl group having 8 to 10 carbon atoms; and a wavy line represents a cis form, a trans form, or a mixture of a cis form and a trans form, examples of the alkyl group having 8 to 10 carbon atoms represented by R include a linear or branched alkyl group, and examples thereof include an octyl group, a nonyl group and a decyl group.

In the structure of the compound of the present invention represented by general formula (1), as shown by two wavy lines, cis and trans isomers are present due to the arrangement of two double bonds. In the present invention, any of cis-cis, cis-trans, trans-cis and trans-trans may be used solely, or a mixture thereof may be used, but preferred is a compound in which position 2 is a trans form and position 4 is a cis form.

(2E)-4-methyl-2,4-tridecadienal, which is one example of the compound of the present invention, can be prepared, for example, by the below-described process.

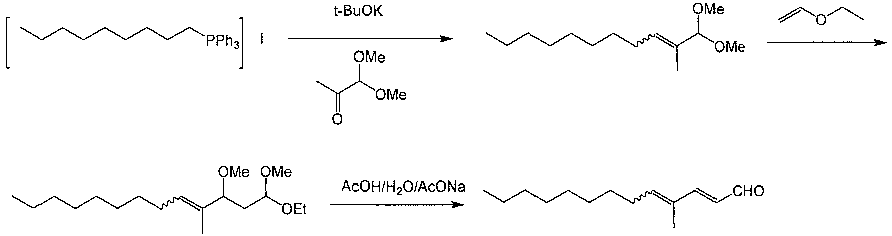

### (1) Synthesis of 2-methyl-2-undecenal dimethyl acetal

Under conditions at 0 to -80°C, to a tetrahydrofuran solution of an ylide prepared using nonyltriphenylphosphonium iodide and potassium t-butoxide, a tetrahydrofuran solution of pyruvic aldehyde dimethyl acetal is added dropwise, and after that, the mixture is stirred until a reaction is completed. After the reaction is completed, it is quenched with water, and then extraction with n-hexane and washing with water are carried out. The reaction solution is concentrated and then distilled, thereby obtaining 2-methyl-2-undecenal dimethyl acetal. Instead of pyruvic aldehyde dimethyl acetal, pyruvic aldehyde diethyl acetal may be used, but preferred is pyruvic aldehyde dimethyl acetal, which is more inexpensive.

### (2) Synthesis of 4-methyl-1,3-dimethoxy-1-ethoxy-4-tridecene

Subsequently, a tetrahydrofuran solution of 2-methyl-2-undecenal dimethyl acetal and boron trifluoride diethyl ether is cooled, and at 3 to 8°C, a tetrahydrofuran solution of ethyl vinyl ether is added dropwise thereto, and after that, the mixture is stirred at the same temperature until a reaction is completed. Boron trifluoride diethyl ether is used in a catalytic amount. Instead of boron trifluoride diethyl ether, zinc chloride or the like may be used. Ethyl vinyl ether is preferably used in a slightly excess amount relative to the amount of 2-methyl-2-undecenal dimethyl acetal.

After the reaction is completed, it is quenched with a sodium bicarbonate solution, and then extraction with diisopropyl ether and washing with water are carried out. The reaction solution is concentrated and then distilled, thereby obtaining 4-methyl-1,3-dimethoxy-1-ethoxy-4-tridecene.

### (3) Synthesis of (2E)-4-methyl-2,4-tridecadienal

Next, a mixture of 4-methyl-1,3-dimethoxy-1-ethoxy-4-tridecene, acetic acid, sodium acetate and water is stirred at 20 to 100°C until a reaction is completed. After the reaction is completed, it is cooled and then extraction with diisopropyl ether is carried out. After that, washing with a sodium bicarbonate solution, and then washing with water are carried out. It is concentrated and then distilled, thereby obtaining (2E)-4-methyl-2,4-tridecadienal. In this regard, acetic acid is preferably used in an amount of 1 to 100 equivalents. Further, sodium acetate is preferably used in an amount of 0.5 to 50 equivalents. Moreover, water is preferably used in an amount of 1 to 100 equivalents. Instead of acetic acid and sodium acetate, formic acid and sodium formate may be used.

In the above-described manner, the intended compound can be obtained.

Note that by using, as a starting material, alkyltriphenylphosphonium iodide or alkyltriphenylphosphonium halide which has a desired alkyl group (R in general formula (1)) instead of nonyltriphenylphosphonium iodide, a dienal compound having another alkyl group (R in general formula (1)) can be obtained. Further, the reaction solvent and the extraction solvent are not limited to the materials exemplified above, and can be suitably selected by those skilled in the art depending on a starting compound, etc.

The dienal compound (1) of the present invention obtained in this way can be purified by means of any of distillation, various chromatographies, etc. Further, a structural isomer can be separated and purified by means of any of various chromatographies, etc.

The dienal compound of the present invention represented by general formula (1) has an excellent aromatic odor, and can be used as a flavor-imparting component for a food or drink, an oral composition, a tobacco product, etc. solely, or in combination with another component. For example, (2E,4Z)-4-methyl-2,4-tridecadienal has a moss-like unique and strong aquarium-like, and a metallic odor, and by adding a suitable amount of it to a citrus-based flavor for beverages, the effect of imparting full-bodied note, juicy, peely and real fruit-like flavor is obtained. Similar effects are also obtained by using an isomer, (2E,4E)-4-methyl-2,4-tridecadienal.

Regarding the flavor composition or dienal compound of the present invention, a slight amount thereof can be directly blended in a food or drink to impart or enhance an aromatic odor/a flavor, and it can also be mixed with another component to impart or enhance an aromatic odor/a flavor of a food or drink. Examples of other flavor components that can be mixed include various synthetic flavors, natural flavors, natural essential oils and plant extracts. For example, the natural essential oils, natural flavors, synthetic flavors, etc. described in "Patent Office Journal, Collection of Well-known Prior Arts (Flavors) Part II, Food Flavors, pp. 88-131, published on January 14, 2000" can be used.

Examples of additives of the flavor composition of the present invention include publicly-known synthetic flavor compounds including:
hydrocarbons such as α-pinene, β-pinene, ocimene, myrcene, limonene, α-phellandrene, terpinene, 3-carene, δ-cadinene, bisabolene and valencene;
alcohols such as 1-hexanol, 1-octanol, 1-nonanol, 1-decanol, 1-undecanol, 2-undecanol, 1-dodecanol, prenol, 10-undecen-1-ol, cis-3-hexenol, linalool, dihydrolinalool, tetrahydromugol, myrcenol, dihydromyrcenol, tetrahydromyrcenol, ocimenol, terpineol, 3-thujanol, benzyl alcohol, β-phenylethyl alcohol and α-phenylethyl alcohol;
aldehydes such as acetaldehyde, n-hexanal, n-heptanal, n-octanal, n-nonanal, 2-methyloctanal, 3,5,5-trimethylhexanal, decanal, undecanal, 2-methyldecanal, dodecanal, tridecanal, tetradecanal, trans-2-hexenal, cis-3-hexenal, trans-2-nonenal, trans-4-decenal, cis-4-decenal, trans-2-decenal, 10-undecenal, trans-2-undecenal, trans-2-dodecenal, 3-dodecenal, trans-2-tridecenal, 2,4-hexadienal, 2,4-nonadienal, 2,4-decadienal, 2,4-dodecadienal, 5,9-dimethyl-4,8-decadienal, citral (neral and geranial), dimethyloctanal, α-methylene citronellal, citronellyl oxyacetaldehyde, myrtenal, α- or β-sinensal, myrac aldehyde and phenyl acetaldehyde;
acetals such as acetaldehyde diethyl acetal, hexanal diethyl acetal, octanal diethyl acetal, nonanal diethyl acetal, decanal diethyl acetal, citral dimethyl acetal, citral diethyl acetal and citral propylene glycol acetal;
ketones such as 3-heptanone, 3-octanone, 2-nonanone, 2-undecanone, 2-tridecanone, methyl heptenone, dimethyl octenone, geranyl acetone, 2,3,5-trimethyl-4-cyclohexenyl-1-methyl ketone, nootkatone, dihydronootkatone, acetophenone, 2,3-butanedione and 4,7-dihydro-2-isopentyl-2-methyl-1,3-dioxepin;
esters such as propyl formate, octyl formate, linalyl formate, citronellyl formate, geranyl formate, neryl formate, terpinyl formate, ethyl acetate, n-propyl acetate, isopropyl acetate, cis-3-hexenyl acetate, trans-2-hexenyl acetate, octyl acetate, nonyl acetate, decyl acetate, dodecyl acetate, dimethyl undecadienyl acetate, ocimenyl acetate, myrcenyl acetate, dihydromyrcenyl acetate, linalyl acetate, citronellyl acetate, geranyl acetate, neryl acetate, terpinyl acetate, citronellyl acetate, nopyl acetate, dihydroterpinyl acetate, decenyl propionate, linalyl propionate, geranyl propionate, neryl propionate, terpinyl propionate, styrallyl propionate, ethyl butyrate, ethyl 2-methylbutyrate, octyl butyrate, neryl butyrate, cinnamyl butyrate, isopropyl isobutyrate, octyl isobutyrate, linalyl isobutyrate, neryl isobutyrate, linalyl isovalerate, terpinyl isovalerate, phenylethyl isovalerate, 2-methylpentyl 2-methylvalerate, methyl 3-hydroxyhexanoate, ethyl 3-hydroxyhexanoate, methyl octanoate, octyl octanoate, linalyl octanoate, methyl nonanoate, methyl undecylenate, linalyl benzoate, methyl cinnamate, isoprenyl angelicate, methyl gelanate and triethyl citrate;
phenols such as thymol, guaiacol, carvacrol and β-naphthol isobutyl ether;
lactones such as γ-decalactone, δ-decalactone, γ-undecalactone and δ-dodecalactone;
carboxylic acids such as acetic acid, propionic acid, butyric acid, valeric acid, hexanoic acid, octanoic acid, decanoic acid, dodecanoic acid, 2-decenoic acid and geranoic acid; and
nitrogen-containing/sulfur-containing compounds such as methyl anthranilate, ethyl anthranilate, methyl N-methyl-anthranilate, methyl N-2'-methyl pentylidene-anthranilate, dodecane nitrile, 2-tridecene nitrile, geranyl nitrile, citronellyl nitrile, 3,7-dimethyl-2,6-nonadienonitrile, indole, 5-methyl-3-heptanone oxime, thiogeraniol, limonene thiol and p-menthen-8-thiol, and products obtained by means of compression, solvent extraction, steam distillation or the like of fruit juice/fruit peel.

The content of the dienal compound in the flavor composition of the present invention varies depending on another flavor component to be mixed and cannot be categorically described, but usually, the concentration can be set within a range of 0.00001 to 10000 ppm, preferably 0.0001 to 1000 ppm, and more preferably 0.001 to 100 ppm based on the mass of the flavor composition.

According to need, the flavor composition of the present invention comprising the dienal compound can contain, for example, a solvent such as water and ethanol; and a fixing agent such as ethylene glycol, propylene glycol, dipropylene glycol, glycerin, hexyl glycol, benzyl benzoate, triethyl citrate, diethyl phthalate, Hercolyn, medium-chain fatty acid triglyceride and medium-chain fatty acid diglyceride, which are usually used.

By the addition of the flavor composition containing the dienal compound of the present invention or the dienal compound itself, an aromatic odor/flavor of various products including a food or drink, an oral composition and a tobacco product can be enhanced.

Specific examples of the food and drink whose aromatic odor/flavor can be enhanced by the addition of the flavor composition or dienal compound of the present invention include: beverages such as carbonated drinks, soft drinks, fruit juice drinks, fruit liquors, milk beverages, lactic fermenting beverages, health drinks, soymilks and tea beverages; desserts such as ice creams, ice milks, lacto-ices, flavored ices, yogurts, puddings, jellies and daily desserts; confectioneries such as caramels, candies, tablet confectioneries, crackers, biscuits, cookies, pies, chocolates, snacks, chewing gums, Manju (steamed buns filled with sweet bean paste) and Yokan (sweet bean jellies); soups such as Japanese-style soups, Western-style soups and Chinese soups; breads; jams; flavor seasonings; various instant drinks; and various instant food products. Among them, as the food and drink whose aromatic odor/flavor can be enhanced by the addition of the flavor composition or dienal compound of the present invention, beverages are preferred, and a citrus-based beverage or beverages are particularly preferred.

The amount of the flavor composition to be added to the food or drink varies depending on the type and form of a product, but the concentration can be set within a range of usually 0.001 to 10% by mass, and preferably 0.01 to 5% by mass based on the mass of the food or drink.

Further, when adding the dienal compound to the food or drink for enhancing an aromatic odor/flavor, the concentration of the dienal compound can be set within a range of 0.00001 to 10000 ppb, preferably 0.0001 to 1000 ppb, and more preferably 0.001 to 100 ppb based on the mass of the food or drink.

Examples of the oral composition to which an aromatic odor/flavor can be imparted by the flavor composition or dienal compound of the present invention include dentifrices, oral cleansers, mouthwashes, troches and chewing gums. The amount of the flavor composition to be added to the oral composition varies depending on the type and form of a product, but usually, it can be set within a range of preferably 0.01 to 5% by mass, and more preferably 0.1 to 3% by mass based on the total mass of the oral composition.

When adding the dienal compound to the oral composition for enhancing an aromatic odor/flavor, the concentration of the dienal compound can be set within a range of 0.00001 to 10000 ppb, preferably 0.0001 to 1000 ppb, and more preferably 0.001 to 100 ppb based on the mass of the oral composition.

Examples of the tobacco product to which an aromatic odor/flavor can be imparted by the flavor composition or dienal compound of the present invention include cigarettes and electronic cigarettes. The amount of the flavor composition to be added to the tobacco product varies depending on the type and form of a product, but for example, it can be set within a range of preferably 0.00001 to 90% by mass, and more preferably 0.0001 to 50% by mass based on the total mass of the portion of tobacco leaves.

When adding the dienal compound to the tobacco product for enhancing an aromatic odor/flavor, the concentration of the dienal compound can be set within a range of 0.00001 to 10000 ppb, preferably 0.0001 to 1000 ppb, and more preferably 0.001 to 100 ppb based on the mass of the tobacco product.

### EXAMPLES

Hereinafter, the present invention will be specifically described based on working examples, but the present invention is not limited thereto.

### [Measurement equipments]

The analytical equipments used in the working examples are as described below.
NMR measurement apparatus: AVANCE III 500 (manufactured by Bruker Biospin)
Gas chromatograph (GC): GC353B (manufactured by GL Sciences Inc.)
Capillary column: Inert CAP (30 m×0.25 mm I.D.)
Column temperature: 80→250°C (temperature raising rate of 10°C/min)
Injection temperature: 200°C
Detector temperature: 250°C
Gas chromatograph mass spectrometer: GCMS-QP2010 (manufactured by Shimadzu Corporation)
Gas chromatograph-Orbitrap mass spectrometer: TRACE1310 (GC)+Exactive GC (mass spectrometer) (manufactured by Thermo Fisher Scientific)
High performance liquid chromatograph (HPLC)
Pump: PU714 (manufactured by GL Sciences Inc.)
Detector: GL-7452A (manufactured by GL Sciences Inc.)
Separation column: COSMOSIL 5SL-II (manufactured by NACALAI TESQUE, INC.)

### (Example 1) Production of (2E)-4-methyl-2,4-tridecadienal

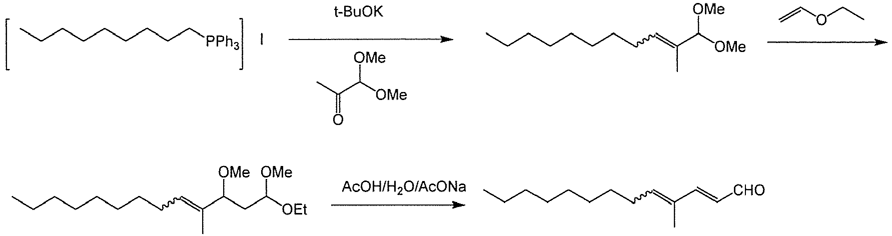

### (1) Synthesis of 2-methyl-2-undecenal dimethyl acetal

To a solution of tetrahydrofuran (500 ml) containing an ylide prepared using 91.41 g (0.177 mol) of nonyltriphenylphosphonium iodide and 23.37 g (0.177 mol) of potassium t-butoxide at -70°C, a solution of tetrahydrofuran (30 ml) containing 23.37 g (0.177 mol) of pyruvic aldehyde dimethyl acetal was added dropwise at -70°C for 1 hour, and after that, the mixture was stirred for 5 hours. After the reaction mixture was quenched with water (250 ml), extraction with n-hexane and washing with water were carried out. After concentration, distillation was carried out (bp. 68°C to 70°C/1 torr), thereby obtaining 26.61 g of 2-methyl-2-undecenal dimethyl acetal (GC purity: 98.6%, 2Z/2E=57/43, yield: 65.8%).
¹H NMR (500 Hz, CDCl₃) δ: 5.44&5.55 (1H, t), 4.44&4.93 (1H, s), 3.29&3.34 (6H, s), 2.04-2.27 (2H, m), 1.59&1.69 (3H, s), 1.22-1.48 (12H, m), 0.88 (3H, t)

### (2) Synthesis of 4-methyl-1,3-dimethoxy-1-ethoxy-4-tridecene

A solution of tetrahydrofuran (40 ml) containing 4.57 g (0.02 mol) of 2-methyl-2-undecenal dimethyl acetal and 0.20 g of boron trifluoride diethyl ether was cooled, and at 3 to 8°C, a solution of tetrahydrofuran (5 ml) containing 2.16 g (0.03 mol) of ethyl vinyl ether was added dropwise thereto over 2 hours, and after that, the mixture was stirred at the same temperature for 2 hours. The reaction mixture was quenched with a sodium bicarbonate solution, and then extraction with diisopropyl ether and washing with water were carried out. After concentration, distillation was carried out (bp. 83°C to 87°C/1 torr), thereby obtaining 4.40 g of 4-methyl-1,3-dimethoxy-1-ethoxy-4-tridecene (yield: 73.2%).
¹H NMR (500 Hz, CDCl₃) δ: 4.50-4.55 (1H, t), 4.17-4.50 (1H, m), 3.42-3.68 (3H, m), 3.31 (3H, s), 3.25 (3H, s), 1.89-2.05 (2H, m), 1.60-1.71 (2H, m), 1.53 (3H, d), 1.19-1.36 (15H, m), 0.88 (3H, t)

### (3) Synthesis of (2E)-4-methyl-2,4-tridecadienal

A mixture of 4-methyl-1,3-dimethoxy-1-ethoxy-4-tridecene (4.20 g (0.014 mol)), acetic acid (30 ml), sodium acetate (2.87 g) and water (7 ml) was stirred at 60°C for 7 hours. The reaction mixture was cooled and then extraction with diisopropyl ether was carried out. After that, washing with a sodium bicarbonate solution, and then washing with water were carried out. After concentration, distillation was carried out (bp. 85°C to 87°C/1 torr), thereby obtaining 2.49 g of (2E)-4-methyl-2,4-tridecadienal (GC purity: 97.8%, 4Z/4E=55/45, yield: 85.3%).

### (Example 2)

(2E)-4-methyl-2,4-tridecadienal synthesized in Example 1 was subjected to purification by means of HPLC (eluant; hexane: ethyl acetate= 97:3 (volume ratio)), thereby obtaining (2E,4Z)-4-methyl-2,4-tridecadienal (purity: 100%) and (2E,4E)-4-methyl-2,4-tridecadienal (purity: 100%).

### <Physical data of (2E,4Z)-4-methyl-2,4-tridecadienal>

¹H NMR (500 MHz, CDCl₃) δ:
9.64 (1H, d, J=7.9 Hz), 7.57 (1H, d, J=15.5 Hz), 6.17 (1H, dd, J=15.5, 7.8 Hz), 5.88 (1H, t, J=7.8 Hz), 2.30 (2H, q, J=7.8 Hz), 1.89 (3H, d, J=1.2 Hz), 1.40-1.47 (2H, m), 1.20-1.35 (10H, m), 0.88 (3H, t, J=7.10 Hz)
¹³C NMR (125 MHz, CDCl₃) δ:
194.42, 148.87, 142.25, 131.12, 128.73, 31.84, 29.53, 29.40, 29.26, 29.22, 28.16, 22.64, 20.03, 14.08
HRMS (EI+): calcd C₁₄H₂₄O₁(M⁺) 208.1822; found, 208.1821

### <Physical data of (2E,4E)-4-methyl-2,4-tridecadienal>

¹H NMR (500 MHz, CDCl₃) δ:
9.56 (1H, d, J=7.9 Hz), 7.12 (1H, d, J=15.6 Hz), 6.10 (1H, dd, J=15.6, 7.9 Hz), 6.04 (1H, t, J=7.7 Hz), 2.24 (2H, q, J=7.5 Hz), 1.81 (3H, s), 1.42-1.48 (2H, m), 1.21-1.36 (10H, m), 0.89 (3H, t, J=7.10 Hz)
¹³C NMR (125 MHz, CDCl₃) δ:
194.22, 157.97, 144.98, 133.36, 126.65, 31.83, 29.41, 29.35, 29.22, 29.07, 28.90, 22.64, 14.07, 12.26
HRMS (EI⁺): calcd C₁₄H₂₄O₁(M⁺) 208.1822; found, 208.1822

### (Example 3) Synthesis of (2E)-4-methyl-2,4-tetradecadienal

Synthesis was performed using decyltriphenylphosphonium iodide instead of nonyltriphenylphosphonium iodide of Example 1, and with bp. 105°C to 108°C/1 torr, (2E)-4-methyl-2,4-tetradecadienal (GC purity: 97.0%, 4Z/4E=57/43) was obtained.

### <Physical data of (2E)-4-methyl-2,4-tetradecadienal>

Regarding letters Z and E in parentheses, Z indicates a signal derived from a (4Z)-isomer and E indicates a signal derived from a (4E)isomer.
¹H NMR (500 MHz, CDCl₃) δ:
9.64 (d, J=7.80 Hz, Z), 9.56 (d, J=7.85 Hz, E), 7.57 (d, J=15.53 Hz, Z), 7.12 (d, J=15.55 Hz, E), 6.17 (dd, J=15.53, 7.80 Hz, Z), 6.10 (dd, J=15.55, 7.85 Hz, E), 6.04 (t, J=7.50 Hz, E), 5.88 (t, J=7.80 Hz, Z), 2.30 (dd, J=7.80 Hz, Z), 2.24 (dd, J=7.50 Hz, E), 1.89 (s, Z), 1.81 (s, E), 1.38-1.50 (m, Z&E), 1.19-1.37 (m, Z&E), 0.88 (t, J=6.88 Hz, Z&E) ¹³C NMR (125 MHz, CDCl₃) δ:
194.40 (Z), 194.20 (E), 157.95 (E), 148.86 (Z), 144.96 (E), 142.24 (Z), 133.35 (E), 131.11 (Z), 128.71 (Z), 126.64 (E), 31.85 (E or Z), 29.52 (E or Z), 29.51 (E or Z), 29.44 (E or Z), 29.34 (E or Z), 29.27 (E or Z), 29.25 (E or Z), 29.07 (E or Z), 28.90 (E or Z), 28.15 (E or Z), 22.64 (E or Z), 20.01 (Z), 14.08 (E&Z), 12.25 (E)
HRMS (CI+): calcd C₁₅H₂₇O₁([M+H]+) 223.2056; found, 223.2057 (Z), 223.2058 (E)

### (Example 4) Evaluation of aromatic odors of synthesized dienals

Regarding (2E,4Z)-4-methyl-2,4-tridecadienal, (2E,4E)-4-methyl-2,4-tridecadienal and (2E)-4-methyl-2,4-tetradecadienal obtained in Examples 1, 2 and 3, evaluation of aromatic odors thereof was made by 6 expert panelists, using flavored water whose concentrations were suitably adjusted. The results are shown in Table 1.

**Table 1: Evaluation results regarding aromatic odors of dienals**

| Compound | Concentration in water | Evaluation of aromatic odor |
|---|---|---|
| (2E,4Z)-4-methyl-2,4-tridecadienal | 0.1 ppb | moss-like strong aquarium-like, and a metallic odor |
| (2E,4E)-4-methyl-2,4-tridecadienal | 0.1 ppb | moss-like, aquarium-like, and metallic, slight oily and fatty odor |
| (2E)-4-methyl-2,4-tetradecadienal | 1 ppb | seaweeds-like, aquarium-like odor |

### (Example 5) Flavor composition for grapefruit food products

Using (2E)-4-methyl-2,4-tridecadienal and (2E)-4-methyl-2,4-tetradecadienal produced by the methods described in Examples 1, 2 and 3, flavor compositions (A-1), (A-2) and (A-3) for grapefruit food products were prepared according to the below-described formulations.

**Formulation of flavor composition (A-1)**

| (Component) | (Parts by mass) |
|---|---|
| (2E,4Z)-4-methyl-2,4-tridecadienal | 0.0001 |
| Grapefruit oil (cold pressed) | 50.0 |
| Ethanol | Balance |
| Total | 1000.0 |

**Formulation of flavor composition (A-2)**

| (Component) | (Parts by mass) |
|---|---|
| (2E,4E)-4-methyl-2,4-tridecadienal | 0.0001 |
| Grapefruit oil (cold pressed) | 50.0 |
| Ethanol | Balance |
| Total | 1000.0 |

**Formulation of flavor composition (A-3)**

| (Component) | (Parts by mass) |
|---|---|
| (2E)-4-methyl-2,4-tetradecadienal | 0.002 |
| Grapefruit oil (cold pressed) | 50.0 |
| Ethanol | Balance |
| Total | 1000.0 |

### (Comparative Example 1)

As Comparative Example 1, a flavor composition (B) for grapefruit food products was prepared according to the below-described formulation.

**Formulation of flavor composition (B)**

| (Component) | (Parts by mass) |
|---|---|
| Grapefruit oil (cold pressed) | 50.0 |
| Ethanol | Balance |
| Total | 1000.0 |

### (Comparative Experiment Example 1)

The flavor compositions (A-1), (A-2) and (B) for grapefruit food products obtained in Example 5 and Comparative Example 1 were respectively added to water in an amount of 0.1% by mass, and a sensory test thereof was conducted by 6 expert panelists. All the expert panelists pointed out that citrus-like juicy and full-bodied and pulpy flavor were imparted by the flavor compositions (A-1) and (A-2) more than the flavor composition (B) and that the flavor compositions (A-1) and (A-2) are remarkably excellent. The same results were obtained in the case of using a mixture of the (2E,4Z)-isomer and the (2E,4E)-isomer.

Regarding the flavor composition (A-3), wherein (2E)-4-methyl-2,4-tetradecadienal was used, and the flavor composition (B), a sensory test was conducted in the same manner. All the expert panelists pointed out that citrus-like peely and full-bodied flavor were imparted by the flavor composition (A-3) more than the flavor composition (B) and that the flavor composition (A-3) is remarkably excellent.

### (Example 6) Addition to commercially available grapefruit juice beverage

0.1 ppb of (2E)-4-methyl-2,4-tridecadienal was added to a commercially available grapefruit juice beverage, and a sensory test thereof was conducted by 6 expert panelists. All the expert panelists pointed out that full-bodied, juicy, and natural fruity flavor were imparted by the addition of (2E)-4-methyl-2,4-tridecadienal and that it is remarkably excellent. The same results were obtained in the case of using the (2E,4Z)-isomer and the (2E,4E)-isomer.

### (Example 7) Addition to commercially available grapefruit juice beverage

2 ppb of (2E)-4-methyl-2,4-tetradecadienal was added to a commercially available grapefruit juice beverage, and a sensory test thereof was conducted by 6 expert panelists. All the expert panelists pointed out that citrus-like peely and full-bodied flavor were imparted by the addition of (2E)-4-methyl-2,4-tetradecadienal and that it is remarkably excellent.

### (Example 8) Flavor composition for grapefruit food products

Flavor compositions (C-1), (C-2) and (C-3) for grapefruit food products were prepared according to the below-described formulations.

**Formulation of flavor composition (C-1)**

| (Component) | (Parts by mass) |
|---|---|
| (2E,4Z)-4-methyl-2,4-tridecadienal | 0.0001 |
| Nootkatone | 2.0 |
| Octanal | 0.3 |
| Decanal | 0.2 |
| Dodecanal | 0.1 |
| Linalool | 0.8 |
| Cis-3-hexenol | 0.5 |
| Ethanol | Balance |
| Total | 1000.0 |

**Formulation of flavor composition (C-2)**

| (Component) | (Parts by mass) |
|---|---|
| (2E,4E)-4-methyl-2,4-tridecadienal | 0.0001 |
| Nootkatone | 2.0 |
| Octanal | 0.3 |
| Decanal | 0.2 |
| Dodecanal | 0.1 |
| Linalool | 0.8 |
| Cis-3-hexenol | 0.5 |
| Ethanol | Balance |
| Total | 1000.0 |

**Formulation of flavor composition (C-3)**

| (Component) | (Parts by mass) |
|---|---|
| (2E)-4-methyl-2,4-tetradecadienal | 0.002 |
| Nootkatone | 2.0 |
| Octanal | 0.3 |
| Decanal | 0.2 |
| Dodecanal | 0.1 |
| Linalool | 0.8 |
| Cis-3-hexenol | 0.5 |
| Ethanol | Balance |
| Total | 1000.0 |

### (Comparative Example 2)

As Comparative Example 2, a flavor composition (D) for grapefruit food products was prepared according to the below-described formulation.

**Formulation of flavor composition (D)**

| (Component) | (Parts by mass) |
|---|---|
| Nootkatone | 2.0 |
| Octanal | 0.3 |
| Decanal | 0.2 |
| Dodecanal | 0.1 |
| Linalool | 0.8 |
| Cis-3-hexenol | 0.5 |
| Ethanol | Balance |
| Total | 1000.0 |

### (Comparative Experiment Example 2)

The flavor compositions (C-1), (C-2) and (D) for grapefruit food products obtained in Example 8 and Comparative Example 2 were respectively added to water in an amount of 0.1% by mass, and a sensory test thereof was conducted by 6 expert panelists. All the expert panelists pointed out that citrus-like juicy and full-bodied and pulpy flavor were imparted by the flavor compositions (C-1) and (C-2) more than the flavor composition (D) and that the flavor compositions (C-1) and (C-2) are remarkably excellent. The same results were obtained in the case of using a mixture of the (2E,4Z)-isomer and the (2E,4E)-isomer.

Regarding the flavor composition (C-3), wherein (2E)-4-methyl-2,4-tetradecadienal was used, and the flavor composition (D), a sensory test was conducted in the same manner. All the expert panelists pointed out that citrus-like peely and full-bodied flavor were imparted by the flavor composition (C-3) more than the flavor composition (D) and that the flavor composition (C-3) is remarkably excellent.

### (Example 9) Flavor composition for orange food products

Flavor compositions (E-1), (E-2) and (E-3) for orange food products were prepared according to the below-described formulations.

**Formulation of flavor composition (E-1)**

| (Component) | (Parts by mass) |
|---|---|
| (2E,4Z)-4-methyl-2,4-tridecadienal | 0.0001 |
| Orange oil (cold pressed) | 50.0 |
| Ethanol | Balance |
| Total | 1000.0 |

### Formulation of flavor composition (E-2)

| (Component) | (Parts by mass) |
|---|---|
| (2E,4E)-4-methyl-2,4-tridecadienal | 0.0001 |
| Orange oil (cold pressed) | 50.0 |
| Ethanol | Balance |
| Total | 1000.0 |

**Formulation of flavor composition (E-3)**

| (Component) | (Parts by mass) |
|---|---|
| (2E)-4-methyl-2,4-tetradecadienal | 0.002 |
| Orange oil (cold pressed) | 50.0 |
| Ethanol | Balance |
| Total | 1000.0 |

### (Comparative Example 3)

As Comparative Example 3, a flavor composition (F) for orange food products was prepared according to the below-described formulation.

**Formulation of flavor composition (F)**

| (Component) | (Parts by mass) |
|---|---|
| Orange oil (cold pressed) | 50.0 |
| Ethanol | Balance |
| Total | 1000.0 |

### (Comparative Experiment Example 3)

The flavor compositions (E-1), (E-2) and (F) for orange food products obtained in Example 9 and Comparative Example 3 were respectively added to water in an amount of 0.1% by mass, and a sensory test thereof was conducted by 6 expert panelists. All the expert panelists pointed out that citrus-like peely, full-bodied and fruity flavor were imparted by the flavor compositions (E-1) and (E-2) more than the flavor composition (F) and that the flavor compositions (E-1) and (E-2) are remarkably excellent. The same results were obtained in the case of using a mixture of the (2E,4Z)-isomer and the (2E,4E)-isomer.

Regarding the flavor composition (E-3), wherein (2E)-4-methyl-2,4-tetradecadienal was used, and the flavor composition (F), a sensory test was conducted in the same manner. All the expert panelists pointed out that citrus-like peely and full-bodied flavor were imparted by the flavor composition (E-3) more than the flavor composition (F) and that the flavor composition (E-3) is remarkably excellent.

### (Example 10) Addition to commercially available orange juice beverage

0.1 ppb of (2E)-4-methyl-2,4-tridecadienal was added to a commercially available orange juice beverage, and a sensory test thereof was conducted by 6 expert panelists. All the expert panelists pointed out that citrus-like peely, full-bodied and fruity flavor were imparted by the addition of (2E)-4-methyl-2,4-tridecadienal and that it is remarkably excellent. The same results were obtained in the case of using the (2E,4Z)-isomer and the (2E,4E)-isomer.

### (Example 11) Addition to commercially available orange juice beverage

2 ppb of (2E)-4-methyl-2,4-tetradecadienal was added to a commercially available orange juice beverage, and a sensory test thereof was conducted by 6 expert panelists. All the expert panelists pointed out that citrus-like peely and full-bodied flavor were imparted by the addition of (2E)-4-methyl-2,4-tetradecadienal and that it is remarkably excellent.

### (Example 12) Flavor composition for orange food products

Flavor compositions (G-1), (G-2) and (G-3) for orange food products were prepared according to the below-described formulations.

**Formulation of flavor composition (G-1)**

| (Component) | (Parts by mass) |
|---|---|
| (2E,4Z)-4-methyl-2,4-tridecadienal | 0.0001 |
| Ethyl butyrate | 0.5 |
| Octanal | 1.0 |
| Nonanal | 0.1 |
| Decanal | 0.5 |
| Linalool | 2.0 |
| Ethanol | Balance |
| Total | 1000.0 |

**Formulation of flavor composition (G-2)**

| (Component) | (Parts by mass) |
|---|---|
| (2E,4E)-4-methyl-2,4-tridecadienal | 0.0001 |
| Ethyl butyrate | 0.5 |
| Octanal | 1.0 |
| Nonanal | 0.1 |
| Decanal | 0.5 |
| Linalool | 2.0 |
| Ethanol | Balance |
| Total | 1000.0 |

**Formulation of flavor composition (G-3)**

| (Component) | (Parts by mass) |
|---|---|
| (2E)-4-methyl-2,4-tetradecadienal | 0.002 |
| Ethyl butyrate | 0.5 |
| Octanal | 1.0 |
| Nonanal | 0.1 |
| Decanal | 0.5 |
| Linalool | 2.0 |
| Ethanol | Balance |
| Total | 1000.0 |

### (Comparative Example 4)

As Comparative Example 4, a flavor composition (H) for orange food products was prepared according to the below-described formulation.

**Formulation of flavor composition (H)**

| (Component) | (Parts by mass) |
|---|---|
| Ethyl butyrate | 0.5 |
| Octanal | 1.0 |
| Nonanal | 0.1 |
| Decanal | 0.5 |
| Linalool | 2.0 |
| Ethanol | Balance |
| Total | 1000.0 |

### (Comparative Experiment Example 4)

The flavor compositions (G-1), (G-2) and (H) for orange food products obtained in Example 12 and Comparative Example 4 were respectively added to water in an amount of 0.1% by mass, and a sensory test thereof was conducted by 6 expert panelists. All the expert panelists pointed out that citrus-like peely, full-bodied and fruity flavor were imparted by the flavor compositions (G-1) and (G-2) more than the flavor composition (H) and that the flavor compositions (G-1) and (G-2) are remarkably excellent. The same results were obtained in the case of using a mixture of the (2E,4Z)-isomer and the (2E,4E)-isomer.

Regarding the flavor composition (G-3), wherein (2E)-4-methyl-2,4-tetradecadienal was used, and the flavor composition (H), a sensory test was conducted in the same manner. All the expert panelists pointed out that citrus-like peely and full-bodied flavor were imparted by the flavor composition (G-3) more than the flavor composition (H) and that the flavor composition (G-3) is remarkably excellent.

### (Example 13) Flavor composition for lemon food products

Flavor compositions (1-1), (1-2) and (1-3) for lemon food products were prepared according to the below-described formulations.

**Formulation of flavor composition (1-1)**

| (Component) | (Parts by mass) |
|---|---|
| (2E,4Z)-4-methyl-2,4-tridecadienal | 0.0001 |
| Lemon oil (cold pressed) | 50.0 |
| Ethanol | Balance |
| Total | 1000.0 |

**Formulation of flavor composition (1-2)**

| (Component) | (Parts by mass) |
|---|---|
| (2E,4E)-4-methyl-2,4-tridecadienal | 0.0001 |
| Lemon oil (cold pressed) | 50.0 |
| Ethanol | Balance |
| Total | 1000.0 |

**Formulation of flavor composition (I-3)**

| (Component) | (Parts by mass) |
|---|---|
| (2E)-4-methyl-2,4-tetradecadienal | 0.002 |
| Lemon oil (cold pressed) | 50.0 |
| Ethanol | Balance |
| Total | 1000.0 |

### (Comparative Example 5)

As Comparative Example 5, a flavor composition (J) for lemon food products was prepared according to the below-described formulation.

**Formulation of flavor composition (J)**

| (Component) | (Parts by mass) |
|---|---|
| Lemon oil (cold pressed) | 50.0 |
| Ethanol | Balance |
| Total | 1000.0 |

### (Comparative Experiment Example 5)

The flavor compositions (I-1), (I-2) and (J) for lemon food products obtained in Example 13 and Comparative Example 5 were respectively added to water in an amount of 0.1% by mass, and a sensory test thereof was conducted by 6 expert panelists. All the expert panelists pointed out that citrus-like peely, full-bodied and fruity flavor were imparted by the flavor compositions (1-1) and (1-2) more than the flavor composition (J) and that the flavor compositions (1-1) and (1-2) are remarkably excellent. The same results were obtained in the case of using a mixture of the (2E,4Z)-isomer and the (2E,4E)-isomer.

Regarding the flavor composition (1-3), wherein (2E)-4-methyl-2,4-tetradecadienal was used, and the flavor composition (J), a sensory test was conducted in the same manner. All the expert panelists pointed out that citrus-like peely and full-bodied flavor were imparted by the flavor composition (1-3) more than the flavor composition (J) and that the flavor composition (1-3) is remarkably excellent.

### (Example 14) Addition to commercially available lemon juice beverage

0.1 ppb of 2(E)-4-methyl-2,4-tridecadienal was added to a commercially available lemon juice beverage, and a sensory test thereof was conducted by 6 expert panelists. All the expert panelists pointed out that citrus-like peely, full-bodied and fruity flavor were imparted by the addition of 2(E)-4-methyl-2,4-tridecadienal and that it is remarkably excellent. The same results were obtained in the case of using the (2E,4Z)-isomer and the (2E,4E)-isomer.

### (Example 15) Addition to commercially available lemon juice beverage

2 ppb of (2E)-4-methyl-2,4-tetradecadienal was added to a commercially available lemon juice beverage, and a sensory test thereof was conducted by 6 expert panelists. All the expert panelists pointed out that citrus-like peely and full-bodied flavor were imparted by the addition of (2E)-4-methyl-2,4-tetradecadienal and that it is remarkably excellent.

### (Example 16) Flavor composition for lemon food products

Flavor compositions (K-1), (K-2) and (K-3) for lemon food products were prepared according to the below-described formulations.

**Formulation of flavor composition (K-1)**

| (Component) | (Parts by mass) |
|---|---|
| (2E,4Z)-4-methyl-2,4-tridecadienal | 0.0001 |
| Citral | 3.0 |
| α-terpineol | 1.0 |
| Geraniol | 0.5 |
| Geranyl acetate | 0.5 |
| Neryl acetate | 0.5 |
| Ethanol | Balance |
| Total | 1000.0 |

**Formulation of flavor composition (K-2)**

| (Component) | (Parts by mass) |
|---|---|
| (2E,4E)-4-methyl-2,4-tridecadienal | 0.0001 |
| Citral | 3.0 |
| α-terpineol | 1.0 |
| Geraniol | 0.5 |
| Geranyl acetate | 0.5 |
| Neryl acetate | 0.5 |
| Ethanol | Balance |
| Total | 1000.0 |

**Formulation of flavor composition (K-3)**

| (Component) | (Parts by mass) |
|---|---|
| (2E)-4-methyl-2,4-tetradecadienal | 0.002 |
| Citral | 3.0 |
| α-terpineol | 1.0 |
| Geraniol | 0.5 |
| Geranyl acetate | 0.5 |
| Neryl acetate | 0.5 |
| Ethanol | Balance |
| Total | 1000.0 |

### (Comparative Example 6)

As Comparative Example 6, a flavor composition (L) for lemon food products was prepared according to the below-described formulation.

**Formulation of flavor composition (L)**

| (Component) | (Parts by mass) |
|---|---|
| Citral | 3.0 |
| α-terpineol | 1.0 |
| Geraniol | 0.5 |
| Geranyl acetate | 0.5 |
| Neryl acetate | 0.5 |
| Ethanol | Balance |
| Total | 1000.0 |

### (Comparative Experiment Example 6)

The flavor compositions (K-1), (K-2) and (L) for lemon food products obtained in Example 16 and Comparative Example 6 were respectively added to water in an amount of 0.1% by mass, and a sensory test thereof was conducted by 6 expert panelists. All the expert panelists pointed out that citrus-like peely, full-bodied and fruity flavor were imparted by the flavor compositions (K-1) and (K-2) more than the flavor composition (L) and that the flavor compositions (K-1) and (K-2) are remarkably excellent. The same results were obtained in the case of using a mixture of the (2E,4Z)-isomer and the (2E,4E)-isomer.

Regarding the flavor composition (K-3), wherein (2E)-4-methyl-2,4-tetradecadienal was used, and the flavor composition (L), a sensory test was conducted in the same manner. All the expert panelists pointed out that citrus-like peely and full-bodied flavor were imparted by the flavor composition (K-3) more than the flavor composition (L) and that the flavor composition (K-3) is remarkably excellent.

### INDUSTRIAL APPLICABILITY

According to the present invention, a novel dienal compound and a flavor composition containing the dienal compound, which are useful for use as a compound flavor raw material or the like, are provided, and these are expected to be widely utilized in the fields of food and drink, oral compositions, tobacco products, etc.

## Claims

1. A compound represented by general formula (1): 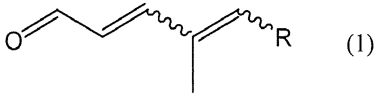 wherein: R represents an alkyl group having 8 to 10 carbon atoms; and a wavy line represents a cis form, a trans form, or a mixture of a cis form and a trans form.

2. The compound of claim 1, wherein R is an alkyl group having 8 carbon atoms.

3. The compound of claim 1 or 2, wherein position 2 is a trans form.

4. The compound of claim 3, wherein position 4 is a cis form.

5. A flavor composition, comprising the compound of any one of claims 1 to 4.

6. The flavor composition of claim 5, wherein the flavor composition is a fruit-like flavor composition.

7. The flavor composition of claim 6, wherein the fruit is a citrus fruit.

8. The flavor composition of claim 6 or 7, wherein the flavor composition is the flavor composition for a food or drink.

9. The flavor composition of claim 8, wherein said food or drink is a beverage.

10. The flavor composition of claim 9, wherein the beverage is a citrus-based beverage.

11. A product selected from the group consisting of a food or drink, an oral composition and a tobacco product, comprising the compound of any one of claims 1 to 4.

12. A product selected from the group consisting of a food or drink, an oral composition and a tobacco product, comprising the flavor composition of any one of claims 5 to 10.
